# EUROPEAN PATENT SPECIFICATION

(11) **EP 3 346 807 B1**
(45) Date of publication and mention of the grant of the patent: **03.06.2020**
(21) Application number: 15903006.3
(22) Date of filing: 02.09.2015
(51) Int. Cl.: H05H 1/24, A61N 1/00, A61N 1/44, A61B 18/04, G06T 7/00

(54) **INFORMATION PROCESSING DEVICE**
INFORMATIONSVERARBEITUNGSVORRICHTUNG
DISPOSITIF DE TRAITEMENT D'INFORMATIONS

(43) Date of publication of application: 11.07.2018
(73) Proprietor: FUJI Corporation, Chiryu Aichi (JP)
(72) Inventor: JINDO, Takahiro, Chiryu, Aichi (JP)
(74) Representative: Grünecker Patent- und Rechtsanwälte PartG mbB
(86) International application number: PCT/JP2015/074920
(87) International publication number: WO 2017/037886

(56) References cited:
- WO-A1-2004/030528
- JP-A- 2005 038 896
- JP-A- 2005 038 896
- JP-A- 2005 116 331
- JP-A- 2012 037 292
- US-A1- 2011 276 113
- US-A1- 2013 199 540
- US-A1- 2014 188 195

## Description

### Technical Field

The present invention relates to an information processing device which is used together with an atmospheric pressure plasma generation device.

### Background Art

In an atmospheric pressure plasma generation device, by ejecting plasma from an ejection port toward a processing target object, plasma is applied to the processing target object such that plasma processing is performed. An example of a plasma generation device is described in PTL 1.

PTL 1: JP-A-2012-059548

### Summary of Invention

### Technical Problem

According to the atmospheric pressure plasma generation device described in PTL 1, it is possible to subject the processing target object to plasma processing. However, since it is not possible to visually check the plasma, there is a concern that it will not be possible to appropriately apply plasma to the processing target object. The present invention is made in consideration of such issues, and an object of the present invention is to appropriately apply plasma to a processing target object.

### Solution to Problem

In order to solve the above problem, an information processing device described in the present application includes an application position calculating section configured to calculate an application position of a plasma on a processing target object based on captured image data of the processing target object when plasma is applied to the processing target object from an atmospheric pressure plasma generation device.

### Advantageous Effects of Invention

In the information processing device described in the present application, the processing target object is imaged by the imaging device when plasma is applied to the processing target object from the atmospheric pressure plasma generation device. The application position of the plasma on the processing target object is calculated by the information processing device based on the captured image data. Accordingly, an operator is able to recognize the application position of the plasma and it is possible to appropriately apply plasma to the processing target object.

### Brief Description of Drawings

[Fig. 1] Fig. 1 is a schematic diagram illustrating a plasma application system.
[Fig. 2] Fig. 2 is an enlarged view illustrating the plasma application device.
[Fig. 3] Fig. 3 is an exploded view illustrating the plasma irradiation device.
[Fig. 4] Fig. 4 is a block diagram illustrating a control device.

### Description of Embodiments

Hereinafter, a detailed description will be given of an example embodiment of the present invention with reference to the drawings.

### Configuration of Plasma Application System

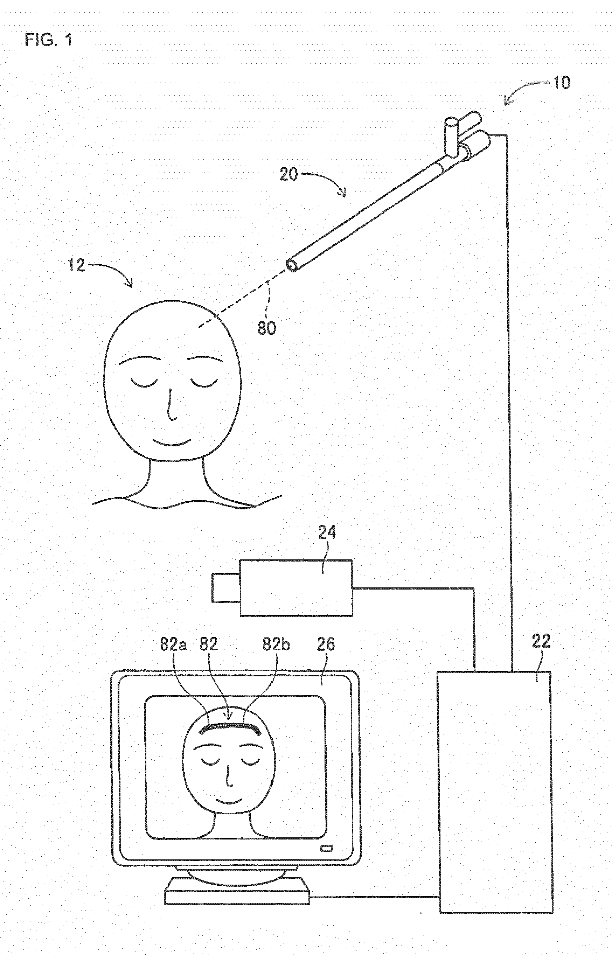

Fig. 1 illustrates plasma application system 10, which is an embodiment of the present invention. Plasma application system 10 is a system that is used in medical applications and is for apply plasma to patient 12. Plasma application system 10 is provided with plasma application device 20, control device 22, imaging device 24, and display device 26.

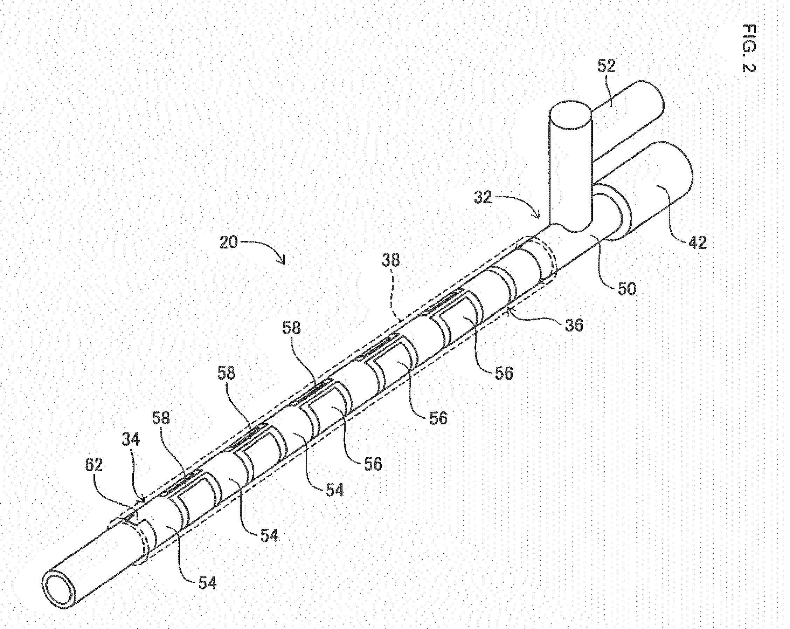

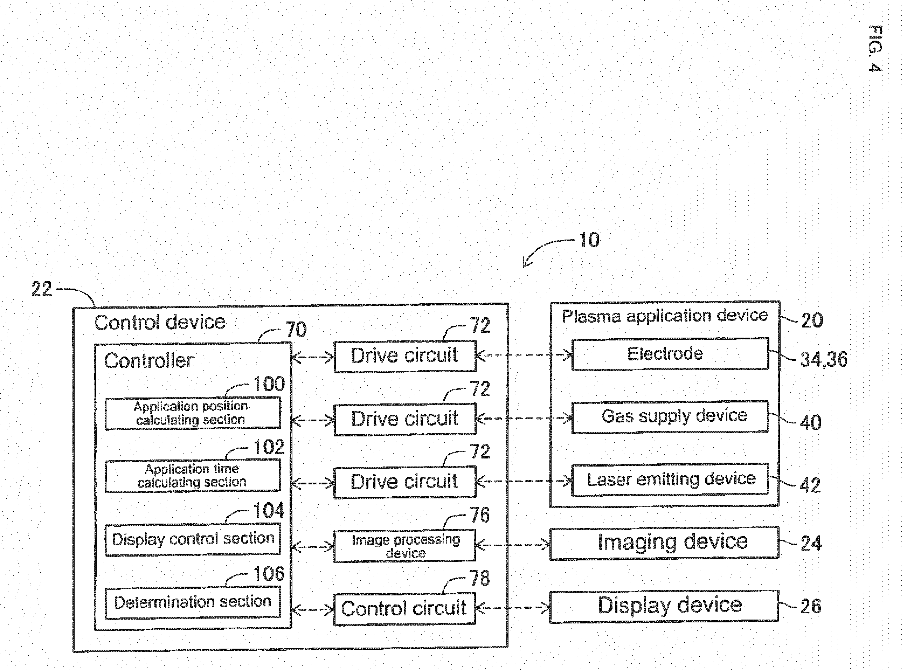

As illustrated in Fig. 2, plasma application device 20 is provided with main body section 32, pair of electrodes 34 and 36, glass tube 38, gas supply device (refer to Fig. 4) 40, and laser emitting device 42.

Main body section 32 is formed of sapphire glass and is configured to include cylindrical section 50 and bent portion 52. Cylindrical section 50 is substantially cylindrical. Bent portion 52 is a tube that is bent into the shape of a letter "L" and one end portion of bent portion 52 is connected in an upright state to an outer circumferential surface close to an end portion of cylindrical section 50. The inner portion of cylindrical section 50 and the inner portion of bent portion 52 are connected.

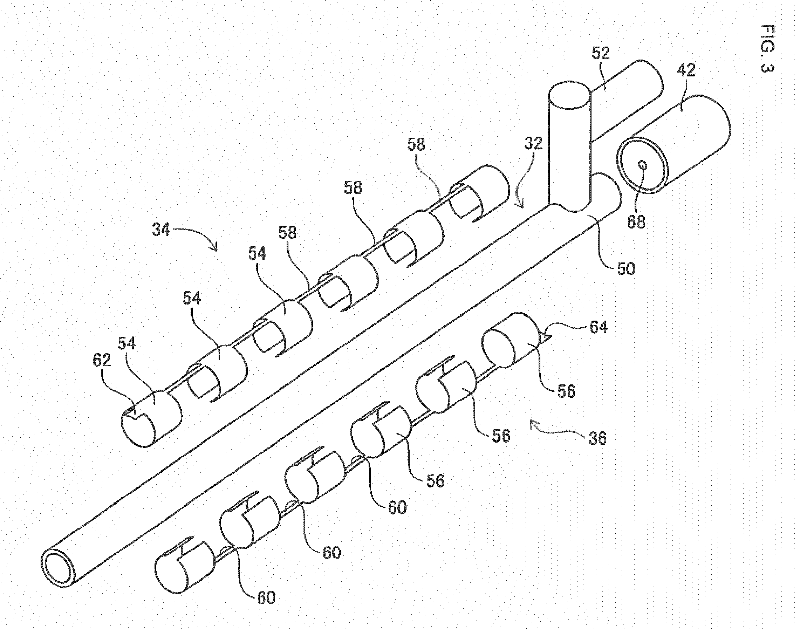

Multiple discharging sections 54 and 56 of the pair of electrodes 34 and 36 are deposited on the outer circumferential surface of cylindrical section 50 of main body section 32 lined up alternately in the axial direction of cylindrical section 50. In detail, as illustrated in Fig. 3, electrode 34 includes multiple discharging sections 54 and multiple connecting sections 58 and electrode 36 includes multiple discharging sections 56 multiple connecting sections 60. Fig. 2 is a virtual view illustrating a state in which electrodes 34 and 36 are removed from cylindrical section 50.

The multiple discharging sections 54 of electrode 34 are deposited on the outer circumferential surface of cylindrical section 50 to extend in the circumferential direction and are installed lined up in the axial direction of cylindrical section 50 at a predetermined interval. Connecting sections 58 of electrode 34 are deposited on the outer circumferential surface of cylindrical section 50 in a linear shape to extend in the axial direction of cylindrical section 50 and connect the multiple discharging sections 54. The discharging section 54 that is positioned at one end of the multiple discharging sections 54 of electrode 34 is deposited along the entire circumference of cylindrical section 50 in the circumferential direction. Meanwhile, the other discharging sections 54 are deposited to extend in the circumferential direction of the cylindrical section 50 excluding the portions on the opposite side from connecting sections 58. Conducting section 62 is formed on discharging section 54 that is deposited along the entire circumference of the circumferential direction of cylindrical section 50 to extend toward the end portion of cylindrical section 50.

The multiple discharging sections 56 of electrode 36 are deposited on the outer circumferential surface of cylindrical section 50 to extend in the circumferential direction and are installed lined up in the axial direction of cylindrical section 50 to be positioned between the multiple discharging sections 54 of electrode 34. Of the multiple discharging sections 56 of electrode 36, discharging sections 56 that are positioned between two discharging sections 54 of electrode 34 are deposited to extend in the circumferential direction of cylindrical section 50 excluding connecting sections 58 of electrode 34. Meanwhile, discharging section 56 that is positioned at the remaining end is deposited along the entire circumference of the circumferential direction of cylindrical section 50. Conducting section 64 is formed on discharging section 56 that is deposited along the entire circumference of the circumferential direction of cylindrical section 50 to extend toward the end portion of cylindrical section 50. Connecting sections 60 of electrode 36 are deposited on the outer circumferential surface of cylindrical section 50 in a linear shape to extend in the axial direction of cylindrical section 50 at locations at which discharging sections 54 of electrode 34 are not deposited and connect the multiple discharging sections 56. In this manner, the pair of electrodes 34 and 36 are deposited on the outer circumferential surface of cylindrical section 50 such that discharging sections 54 of electrode 34 and discharging sections 56 of electrode 36 are lined up alternately at a predetermined interval.

As illustrated in Fig. 2, glass tube 38 is installed on the outer peripheral surface of cylindrical section 50 of main body section 32 and covers the entirety of the pair of electrodes 34 and 36 that are deposited on the outer circumferential surface of cylindrical section 50. Accordingly, it becomes possible to prevent the exposure of electrodes 34 and 36 to which a high voltage is applied and safety is secured. Since electrodes 34 and 36 are sealed by glass tube 38, glass tube 38 enters between discharging sections 54 of electrode 34 and discharging sections 56 of electrode 36.

Gas supply device 40 is a device that supplies processing gas and is connected to the end portion of bent portion 52 on the opposite side to the end portion of bent portion 52 that is connected to cylindrical section 50. Accordingly, processing gas is supplied to the inner portion of cylindrical section 50 via bent portion 52. The processing gas may be a gas in which an inert gas such as nitrogen and an active gas such as the oxygen in the air are combined at a given ratio, or may be only an inert gas or air.

Laser emitting device 42 is a device that emits laser light and is substantially cylindrical and short. An end surface of laser emitting device 42 is connected coaxially to an end surface of cylindrical section 50 at the side on which bent portion 52 is installed. Emission port (refer to Fig. 3) 68 is formed in the center portion of the end surface of laser emitting device 42 that is connected to cylindrical section 50 and laser emitting device 42 emits laser light from emission port 68 in the axial direction of cylindrical section 50. The laser light is red laser light of a high frequency in the visible region.

As illustrated in Fig. 4, control device 22 is provided with controller 70, multiple drive circuits 72, image processing device 76, and control circuit 78. Multiple drive circuits 72 are connected to electrodes 34 and 36, gas supply device 40, and laser emitting device 42. Controller 70 is provided with a CPU, ROM, RAM, and the like, the main constituent of controller 70 is a computer, and controller 70 is connected to the multiple drive circuits 72. Accordingly, the operations of plasma application device 20 are controlled by controller 70. Controller 70 is also connected to image processing device 76. Image processing device 76 is a device for processing captured image data that is captured by imaging device 24. Accordingly, controller 70 acquires data that is obtained using the captured image data. Controller 70 is connected to display device 26 via control circuit 78. Accordingly, controller 70 is capable of causing display device 26 to display any given image.

### Plasma Application by Plasma Application System

According to the above-described configuration, in plasma application system 10, with plasma irradiation device 20, plasma is ejected from the end portion of cylindrical section 50 such that plasma is applied to the processing target object. Plasma includes active oxygen radicals and is utilized in the field of medicine with the object of treating an affected part using the active oxygen radicals. Specifically, for example, since skin is revitalized due to plasma being applied to the skin, plasma is applied with the object of regenerating skin. Since cancerous cells are killed by plasma being applied to them, plasma application is performed with the object of cancer treatment.

Specifically, in plasma irradiation device 20, the processing gas is supplied to the inner portion of cylindrical section 50 via bent portion 52 by gas supply device 40. Since the end surface of cylindrical section 50 on which bent portion 52 is installed is blocked by the laser emitting device 42, the processing gas that is supplied to cylindrical section 50 flows toward the end surface of the opposite side from the end surface on which bent portion 52 is installed. In other words, the processing gas flows toward the inner portion of cylindrical section 50 on which electrodes 34 and 36 are deposited.

A voltage is applied to conducting sections 62 and 64 of electrodes 34 and 36 and a current flows in electrodes 34 and 36. Accordingly, a discharge is generated between discharging sections 54 and 56 of the pair of electrodes 34 and 36. At this time, since electrodes 34 and 36 are sealed by glass tube 38, which is an insulator, discharge is generated in the inner portion of cylindrical section 50 and processing gas that flows in the inner portion of cylindrical section 50 is turned into plasma. The plasma is ejected toward the axial direction of cylindrical section 50 from an opening (hereinafter, the opening may be referred to as an "ejection port") of the end surface of the opposite side from the end surface of cylindrical section 50 on the side to which laser emitting device 42 is connected. Here, by a clinician such as a doctor causing the ejection port of plasma application device 20 to point towards an affected part of patient 12, plasma is applied to the affected part so as to treat the affected part.

However, since the wavelength of the plasma is a wavelength in a vacuum ultraviolet region, it is not possible to visually check the plasma. Therefore, there is a concern that plasma may not be appropriately applied to the affected part. In consideration of this, laser emitting device 42 is provided on plasma irradiation device 20 and it is possible to check the application position of the plasma using laser light that is emitted by laser emitting device 42.

In detail, as described above, laser emitting device 42 is connected to the end surface of cylindrical section 50 and emits laser light in the axial direction of cylindrical section 50. In other words, laser emitting device 42 emits laser light coaxially with the ejection direction of the plasma. Laser light is straight parallel light. Therefore, the laser light that is emitted from laser emitting device 42 passes through the inside of cylindrical section 50 and is emitted from the ejection port of cylindrical section 50 coaxially with the ejection direction of the plasma. Accordingly, as illustrated in Fig. 1, laser light 80 irradiates the location to which the plasma is applied. Since the laser light is red laser light of a high frequency in the visible region, the clinician is able to appropriately apply plasma to the affected part by visually checking the laser light.

Plasma application by plasma application device 20 is performed after checking the application position using the laser light. In detail, first, laser light 80 is emitted by laser emitting device 42. Here, the supplying of the processing gas by gas supply device 40 and application of a voltage to electrodes 34 and 36 are not performed. An operator aligns laser light 80 with the planned plasma application position by causing the ejection port of cylindrical section 50 to point towards the affected part. Once laser light 80 is aligned to the planned plasma application position, the processing gas is supplied by gas supply device 40 and the voltage is applied to electrodes 34 and 36. Accordingly, it becomes possible to appropriately apply plasma to the planned application position.

In plasma application system 10, when plasma is applied to the affected part, the affected part is imaged by imaging device 24 and an image based on the captured image data is displayed on display device 26 together with the application position of the plasma. In detail, while plasma is being applied to the affected part, the affected part is imaged by imaging device 24 and the captured image data is transmitted to controller 70 of control device 22. Controller 70 calculates the application position of the laser light, that is, the application position of the plasma based on the captured image data. Since the laser light with which the affected part is irradiated is a red laser light and red is a color that is easy to analyze, it is possible to favorably calculate the application position of the plasma.

When the application position of the plasma is calculated, in controller 70, image data in which an image based on the captured image data of imaging device 24, that is, an image of the patient to whom plasma is to be applied, and an image corresponding to the calculated application position are overlaid is created. The created image data is transmitted to display device 26 and an image based on the image data is displayed on display device 26. As illustrated in Fig. 1, plasma application position 82 that has been applied to the affected part of the patient is displayed in the image. Accordingly, the clinician is able to perform treatment while checking the locations for which plasma application is complete.

In plasma application system 10, the display mode of plasma application position 82 differs according to the plasma application time. In detail, in controller 70, when the application position of the plasma is calculated, the application time of the laser light per unit area of the application position, that is, the application time of the plasma is also calculated. When creating the image data in which the image of the patient and the image of plasma application position 82 are overlaid, the image of plasma application position 82 is rendered darker the longer the application time per unit area is and lighter the shorter the application time per unit area is. Therefore, when the image based on the image data is displayed on display device 26, as illustrated in Fig. 1, plasma application position 82 is displayed in display mode 82a in the light state and display mode 82b in the dark state. Accordingly, the clinician is able to perform treatment while checking the application amount of the plasma.

In plasma application system 10, excessive application of plasma is prevented. In detail, as described above, in controller 70, when the application position of the plasma is calculated, the application time of the plasma per unit area of the application position is also calculated. At this time, in controller 70, it is determined whether the calculated application time of the plasma per unit area exceeds a set time that is set in advance. In a case in which it is determined that the application time of the plasma per unit area exceeds the set time, the power supply to electrodes 34 and 36 of plasma application device 20 is cut off. Accordingly, when the plasma application amount to a specific part exceeds a predetermined amount, the application of plasma is stopped, making it possible to prevent excessive application of plasma to a specific part.

The calculated application position and the calculated application time per unit area of the plasma are stored for each patient in control device 22. Accordingly, the plasma application position and the plasma application amount per unit area of the application position are managed for each patient and it is possible to effectively perform treatment.

As illustrated in Fig. 4, controller 70 of control device 22 includes application position calculating section 100, application time calculating section 102, display control section 104, and determination section 106. Application position calculating section 100 is a functional unit for calculating plasma application position 82 based on the captured image data. Application time calculating section 102 is a functional unit for calculating the application time of the plasma based on the captured image data. Display control section 104 is a functional unit for causing display device 26 to display an image in which the patient and plasma application position 82 are overlaid. Determination section 106 is a functional unit for determining whether the application time per unit area of the plasma exceeds the set time.

Note that, plasma irradiation device 20 is an example of an atmospheric pressure plasma generation device. Control device 22 is an example of an information processing device. Display device 26 is an example of a display device. Laser emitting device 42 is an example of an irradiation device. Application position calculating section 100 is an example of an application position calculating section. Application time calculating section 102 is an example of an application time calculating section. Display control section 104 is an example of a display control section. Determination section 106 is an example of a determination section.

The present invention is not limited to the above embodiment and it is possible to carry out the present invention in various modes subjected to various modifications and improvements based on the knowledge of a person skilled in the art. Specifically, for example, in the example, the clinician holds plasma application device 20 to apply plasma to the affected part; however, it is possible to operate plasma application device 20 using a robot hand or the like to apply plasma to the affected part. When operating plasma application device 20 using the robot hand or the like, it is possible to appropriately apply plasma to the affected part by using plasma application position 82 that is calculated by controller 70.

In an embodiment above, the shading of the image of the application position of the plasma is changed according to the application time of the plasma; however, it possible to change the color of the image. Specifically, for example, it is possible to display an image of the application position of the plasma in a red-based color the longer the application time of the plasma is and to display an image of the application position of the plasma in a blue-based color the shorter the application time of the plasma is.

In an embodiment above, in a case in which the application time of the plasma exceeds the set time, the application of the plasma is stopped; however, the clinician may be notified of the fact that the application time of the plasma has exceeded the set time using sound, light, or the like.

In an embodiment above, an image of the application position of the plasma is displayed on display device 26 such as a monitor; however, it is possible to display the application position of the plasma on an eyeglasses-form display device such as Google Glass (registered trademark). Here, only the application position of the plasma is displayed on the eyeglasses-form display device and the clinician visually recognizes the actual patient in a state with the application position of the plasma which is displayed on the eyeglasses-form display device overlaid.

In an embodiment above, plasma application device 20 is provided with laser emitting device 42 and the application position of the laser light is calculated as the application position of the plasma by control device 22; however, it is possible to calculate the application position of the plasma itself using control device 22 without providing plasma application device 20 with laser emitting device 42. In detail, for example, a special imaging device capable of identifying light of a wavelength in the vacuum ultraviolet region may be adopted as imaging device 24 . Accordingly, it becomes possible to calculate the application position of the plasma based on the captured image data of the special imaging device.

In an embodiment above, plasma application system 10 is used in the field of medicine; however, it is possible to use plasma application system 10 in various fields such as an industrial field. In an industrial field, for example, it is possible to use plasma application system 10 with the object of surface treatment, surface improvement, and the like of metals and the like.

### Reference Signs List

20: plasma irradiation device (atmospheric pressure plasma generation device), 22: control device (information processing device), 26: display device, 42: laser irradiation device (irradiation device), 100: application position calculating section, 102: irradiation time calculating section, 104: display control section, 106: determination

Documents WO-A-2004/030528, US-A-2011/0276113 and US-A-2013/0199540 describe different plasma application devices.The embodiments, examples or aspects according to the present description which do not fall within the scope of the appended claims are provided for illustrative purposes only and do not form part of the present invention, which is defined by the following claims:

## Claims

1. A system comprising an atmospheric pressure plasma generation device (20), an imaging device (24), and an information processing device (22), the information processing device (22) comprising:
an application position calculating section (100) configured to calculate an application position (82) of plasma on a processing target object (12) based on captured image data of the processing target object (12) captured by the imaging device (24) when plasma is applied to the processing target object (12) from the atmospheric pressure plasma generation device (20).

2. The system according to Claim 1, further comprising:
a display device (26),
wherein the information processing device (22) further comprises a display control section (104) configured to cause the display device (26) to display an image corresponding to the application position (82) of the plasma that is calculated by the application position calculating section (100).

3. The system according to Claim 2,
wherein the information processing device (22) further comprises an application time calculating section (102) configured to calculate an plasma application time on the processing target object (12) based on the captured image data of the processing target object (12) when plasma is applied to the processing target object (12) from the atmospheric pressure plasma generation device (20),
wherein the display control section (104) is configured to display an image corresponding to the application position (82) of the plasma in a state in which a display mode is changed depending on the plasma application time that is calculated by the application time calculating section (102).

4. The system according to Claim 1 or 2,
wherein the information processing device (22) further comprises an application time calculating section (102) configured to calculate an plasma application time on the processing target object (12) based on the captured image data of the processing target object (12) when plasma is applied to the processing target object (12) from the atmospheric pressure plasma generation device (20).

5. The system according to Claim 4,
wherein the information processing device (22) further comprises a determination section (106) configured to determine whether the plasma application time that is calculated by the application time calculating section (102) exceeds a set time.

6. The system according to any one of Claims 1 to 5,
wherein the atmospheric pressure plasma generation device (20) is provided with an ejection port that ejects the plasma and an emitting device (42) that emits light from the ejection port in an ejection direction of the plasma, and
wherein the application position calculating section (100) is configured to calculate the application position (82) of the plasma on the processing target object (12) based on captured image data of light that is emitted from the emitting device (42).

## Patentansprüche

1. System, umfassend eine Vorrichtung (20) zur Erzeugung von Atmosphärendruck-Plasma, eine Bilderfassungsvorrichtung (24) und eine Informationsverarbeitungsvorrichtung (22), wobei die Informationsverarbeitungsvorrichtung (22) umfasst:
einen Anwendungspositions-Berechnungsabschnitt (100), der konfiguriert ist, eine Anwendungsposition (82) von Plasma auf einem Bearbeitungs-Zielobjekt (12) basierend auf aufgenommenen Bilddaten des Bearbeitungs-Zielobjekts (12) zu berechnen, die von der Bilderfassungsvorrichtung (24) aufgenommen werden, wenn von der Vorrichtung (20) zur Erzeugung von Atmosphärendruck-Plasma Plasma auf das Bearbeitungs-Zielobjekt (12) appliziert wird.

2. Das System gemäß Anspruch 1, ferner umfassend:
eine Anzeigevorrichtung (26),
wobei die Informationsverarbeitungsvorrichtung (22) ferner einen Anzeigesteuerungsabschnitt (104) umfasst, der konfiguriert ist, die Anzeigevorrichtung (26) zu veranlassen, ein Bild entsprechend der Anwendungsposition (82) des Plasmas anzuzeigen, die von dem Anwendungspositions-Berechnungsabschnitt (100) berechnet wird.

3. Das System gemäß Anspruch 2,
wobei die Informationsverarbeitungsvorrichtung (22) ferner einen Anwendungszeit-Berechnungsabschnitt (102) umfasst, der konfiguriert ist, eine Plasmaanwendungszeit auf das Bearbeitungs-Zielobjekt (12) basierend auf den aufgenommenen Bilddaten des Bearbeitungs-Zielobjekts (12) zu berechnen, wenn von der Vorrichtung (20) zur Erzeugung von Atmosphärendruck-Plasma Plasma auf das Bearbeitungs-Zielobjekt (12) appliziert wird,
wobei der Anzeigesteuerungsabschnitt (104) konfiguriert ist, ein Bild entsprechend der Anwendungsposition (82) des Plasmas in einem Zustand anzuzeigen, in welchem ein Anzeigemodus in Abhängigkeit von der Plasmaanwendungszeit geändert wird, die von dem Anwendungszeit-Berechnungsabschnitt (102) berechnet wird.

4. Das System gemäß Anspruch 1 oder 2,
wobei die Informationsverarbeitungsvorrichtung (22) ferner einen Anwendungszeit-Berechnungsabschnitt (102) umfasst, der konfiguriert ist, eine Plasmaanwendungszeit auf das Bearbeitungs-Zielobjekt (12) basierend auf den aufgenommenen Bilddaten des Bearbeitungs-Zielobjekts (12) zu berechnen, wenn von der Vorrichtung (20) zur Erzeugung von Atmosphärendruck-Plasma Plasma auf das Bearbeitungs-Zielobjekt (12) appliziert wird.

5. Das System gemäß Anspruch 4,
wobei die Informationsverarbeitungsvorrichtung (22) ferner einen Bestimmungsabschnitt (106) umfasst, der konfiguriert ist, zu bestimmen, ob die Plasmaanwendungszeit, die von dem Anwendungszeit-Berechnungsabschnitt (102) berechnet wird, eine festgelegte Zeit überschreitet.

6. Das System gemäß einem der Ansprüche 1 bis 5,
wobei die Vorrichtung (20) zur Erzeugung von Atmosphärendruck-Plasma mit einer Ausstoßöffnung ausgestattet ist, die das Plasma ausstößt und einer Aussendevorrichtung (42), die von der Ausstoßöffnung Licht in einer Ausstoßrichtung des Plasmas aussendet, und
wobei der Anwendungspositions-Berechnungsabschnitt (100) konfiguriert ist, die Anwendungsposition (82) des Plasmas auf dem Bearbeitungs-Zielobjekt (12) basierend auf aufgenommenen Bilddaten von Licht zu berechnen, das von der Aussendevorrichtung (42) ausgesendet wird.

## Revendications

1. Système comprenant un dispositif de génération de plasma (20) sous pression atmosphérique, un dispositif de formation en image (24) et un dispositif de traitement d'informations (22), le dispositif de traitement d'informations (22) comprenant :
une section de calcul de position d'application (100) configurée pour calculer la position de l'application (82) du plasma sur un objet cible de traitement (12) sur la base de données d'image prise de l'objet cible de traitement (12), prise par le dispositif de formation en image (24) lorsque le plasma est appliqué sur l'objet cible de traitement (12) à partir du dispositif de génération de plasma (20) sous pression atmosphérique.

2. Système selon la revendication 1, comprenant en outre :
un dispositif d'affichage (26),
dans lequel le dispositif de traitement d'informations (22) comprend en outre une section de commande d'affichage (104) configurée pour amener le dispositif d'affichage (26) à afficher une image correspondant à la position d'application (82) du plasma, laquelle est calculée par la section de calcul de position d'application (100).

3. Système selon la revendication 2,
dans lequel le dispositif de traitement d'informations (22) comprend en outre une section de calcul de durée d'application (102) configurée pour calculer la durée d'application du plasma sur l'objet cible de traitement (12) sur la base des données d'image prise sur l'objet cible de traitement (12) lorsque le plasma est appliqué sur l'objet cible de traitement (12) à partir du dispositif de génération de plasma (20) sous pression atmosphérique,
dans lequel la section de commande d'affichage (104) est configurée pour afficher une image correspondant à la position d'application (82) du plasma de telle sorte que le mode d'affichage soit modifié en fonction de la durée d'application du plasma qui est calculée par la section de calcul de durée d'application (102).

4. Système selon la revendication 1 ou la revendication 2,
dans lequel le dispositif de traitement d'informations (22) comprend en outre une section de calcul de durée d'application (102) configurée pour calculer la durée d'application du plasma sur l'objet cible de traitement (12) sur la base des données d'image prise de l'objet cible de traitement (12) lorsque le plasma est appliqué sur l'objet cible de traitement (12) à partir du dispositif de génération de plasma (20) sous pression atmosphérique.

5. Système selon la revendication 4,
dans lequel le dispositif de traitement d'informations (22) comprend en outre une section de détermination (106) configurée pour déterminer si la durée d'application du plasma qui est calculée par la section de calcul de durée d'application (102) dépasse une durée fixée.

6. Système selon l'une quelconque des revendications 1 à 5,
dans lequel le dispositif de génération de plasma (20) sous pression atmosphérique est muni d'un orifice d'éjection qui éjecte le plasma, ainsi que d'un dispositif d'émission (42) qui émet de la lumière à partir de l'orifice d'éjection dans la direction d'éjection du plasma, et
dans lequel la section de calcul de position d'application (100) est configurée pour calculer la position d'application (82) du plasma sur l'objet cible de traitement (12) sur la base des données d'image prise de la lumière qui est émise par le dispositif d'émission (42).
